(19) Europäisches Patentamt
European Patent Office
Office européen des brevets

(11) **EP 2 857 812 B1**

(12) **EUROPÄISCHE PATENTSCHRIFT**

(45) Veröffentlichungstag und Bekanntmachung des Hinweises auf die Patenterteilung:
**27.04.2016 Patentblatt 2016/17**

(51) Int Cl.:
*G01J 3/433* *(2006.01)*        *G01J 3/28* *(2006.01)*

(21) Anmeldenummer: **13186991.9**

(22) Anmeldetag: **02.10.2013**

(54) **VERFAHREN ZUR MESSUNG DER KONZENTRATION EINER GASKOMPONENTE IN EINEM MESSGAS**

METHOD FOR MEASURING THE CONCENTRATION OF A GAS COMPONENT IN A GAS TO BE MEASURED

PROCÉDÉ DE MESURE DE LA CONCENTRATION D'UN COMPOSANT DE GAZ DANS UN GAZ DE MESURE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**08.04.2015 Patentblatt 2015/15**

(73) Patentinhaber: **Siemens Aktiengesellschaft**
**80333 München (DE)**

(72) Erfinder:
- **Bitter, Ralf**
  **76139 Karlsruhe (DE)**
- **Steinbacher, Franz**
  **76137 Karlsruhe (DE)**
- **Hankiewicz, Thomas**
  **76149 Karlsruhe (DE)**
- **Marquardt, Christoph Wolfgang**
  **76149 Karlsruhe (DE)**
- **Nygren, Jan**
  **76137 Karlsruhe (DE)**
- **Pleban, Kai-Uwe**
  **76297 Stutensee (DE)**

(56) Entgegenhaltungen:
**DE-A1- 3 021 041**

- **KLUCZYNSKI P ET AL: "THEORETICAL DESCRIPTION BASED ON FOURIER ANALYSIS OF WAVELENGTH-MODULATION SPECTROMETRY IN TERMS OF ANALYTICAL AND BACKGROUND SIGNALS", APPLIED OPTICS, OPTICAL SOCIETY OF AMERICA, WASHINGTON, DC; US, Bd. 38, Nr. 27, 20. September 1999 (1999-09-20), Seiten 5803-5815, XP001176660, ISSN: 0003-6935, DOI: 10.1364/AO.38.005803**
- **PAWEL KLUCZYNSKI ET AL: "Characterization of Background Signals in Wavelength-Modulation Spectrometry in Terms of a Fourier Based Theoretical Formalism", APPLIED OPTICS, Bd. 40, Nr. 6, 1. Januar 2001 (2001-01-01), Seite 770, XP055093146, ISSN: 0003-6935, DOI: 10.1364/AO.40.000770**

**Beschreibung**

Die Erfindung betrifft ein Verfahren zur Messung der Konzentration einer Gaskomponente in einem Messgas mittels eines Gasanalysators, wobei

- zur wellenlängenabhängigen Abtastung einer interessierenden Absorptionslinie der Gaskomponente die Wellenlänge des Lichts einer wellenlängenabstimmbaren Lichtquelle innerhalb periodisch aufeinanderfolgender Abtastintervalle variiert und dabei zusätzlich mit einer Frequenz moduliert wird,
- das modulierte Licht durch das Messgas auf einen Detektor geführt wird,
- ein von dem Detektor erzeugtes Messsignal bei einer Oberschwingung der Frequenz demoduliert wird, und
- durch Anfitten einer Sollkurve an den Verlauf des demodulierten Messsignals ein Messergebnis erzeugt wird.

Ein derartiges Verfahren ist aus der EP 1 475 618 B1 bekannt.

Bei dem bekannten Verfahren erzeugt eine wellenlängenabstimmbare Lichtquelle in Form einer Laserdiode Licht im Infrarotbereich, das durch ein zu messendes Prozessgas (Messgas) geleitet und anschließend detektiert wird. Die Wellenlänge des Lichts wird auf eine spezifische Absorptionslinie der jeweils zu messenden Gaskomponente abgestimmt, wobei die Absorptionslinie periodisch wellenlängenabhängig abgetastet wird. Dazu wird die Laserdiode innerhalb von periodisch aufeinanderfolgenden Abtastintervallen mit einem rampen- oder dreieckförmigen Stromsignal angesteuert. Während der vergleichsweise langsamen Abtastung der Absorptionslinie wird zusätzlich die Wellenlänge des erzeugten Lichts mit hoher Frequenz und kleiner Amplitude sinusförmig moduliert. Da das Profil der Absorptionslinie nicht linear ist, werden in dem bei der Detektion erhaltenen Messsignal auch Harmonische oberhalb der Modulationsfrequenz erzeugt. Das Messsignal wird üblicherweise bei einer n-ten Oberschwingung, vorzugsweise der zweiten Harmonischen, durch phasensensitive Lock-in Technik demoduliert und für jedes Abtastintervall zu einem Messergebnis ausgewertet. Bei kleiner Modulationsamplitude ist die Detektion der n-ten Harmonischen direkt proportional zu der n-ten Ableitung des direkten Messsignals. Die Auswertung erfolgt z. B. durch Anfitten (Curve-Fitting) des im Idealfall zu erwartenden Verlaufs des demodulierten Messsignals (Sollkurve) an dessen tatsächlichen Verlauf (Istkurve). Aus dem dabei erhaltenen Messergebnis wird schließlich die Konzentration der zu messenden Gaskomponente bestimmt.

Temperaturänderungen innerhalb des Gasanalysators können zu Änderungen der Messergebnisse führen. Diese als Drift bezeichnete Charakteristik des Gasanalysators schränkt sein Messverhalten und zu realisierende Applikationen maßgeblich ein. Eine Ursache für die Drift können unter anderem Etalons im optischen Strahlengang sein. Diese führen in dem Verlauf des demodulierten Messsignals zu periodischen Strukturen, die im Frequenzbereich des zu erwartenden Absorptionssignals liegen. Beim Curve-Fitting führt dies zu fehlangefitteten Funktionen und Abweichungen zwischen der ermittelten Konzentrationen von der tatsächlichen Konzentration der zu messenden Gaskomponente.

Zur Unterdrückung dieser Störsignalanteile ist es aus der oben genannten EP 1 475 618 B1 bekannt, einen Teil des von der Lichtquelle erzeugten Lichts unmittelbar auf einen Monitordetektor zu führen und das erhaltene Monitorsignal bei der n-ten Oberschwingung zu demoduliert und auszuwerten. Jede Abweichung des demodulierten Monitorsignals von einer Nulllinie beruht auf einer optischen Störung, die, soweit sie im Bereich der Lichtquelle oder in dem von Mess- und Monitorkanal gemeinsamen genutzten Wegabschnitt des Strahlengangs liegt, auch das Messsignal beeinträchtigt. Diese Störung wird durch eine Vorverzerrung der Ansteuerung der Lichtquelle kompensiert, indem die Wellenlänge des Lichts zusätzlich mit der n-ten Oberschwingung moduliert wird, wobei die Modulationsintensität von dem demodulierten Monitorsignal abhängig ist. Die Auskopplung eines Teils des erzeugten Lichts auf den Monitordetektor ist jedoch mit einem erhöhten konstruktiven und schaltungstechnischen Aufwand verbunden, der mit einer höheren Störempfindlichkeit einhergeht. Außerdem können außerhalb der gemeinsamen Abschnitte des Mess- und Monitorkanals auftretende Störungen des Messsignals nicht kompensiert werden.

Aus der EP 2 336 738 A1 oder EP 1 927 831 A1 ist es bekannt, die optische Weglänge beispielsweise durch mechanische Vibration der Lichtquelle zu variieren und die störenden periodischen Strukturen aus dem demodulierten Messsignal herauszumitteln. Dadurch lassen sich aber nur bestimmte, von parallelen optischen Oberflächen im Stahlengang erzeugte Interferenz-Störungen reduzieren.

Der Erfindung liegt die Aufgabe zugrunde, aus Störeinflüssen wie z. B. Temperaturänderungen in dem Gasanalysator resultierende Änderungen in den Messergebnissen zu reduzieren.

Gemäß der Erfindung wird die Aufgabe dadurch gelöst, dass bei dem Verfahren der eingangs angegebenen Art

- eine zu der Sollkurve orthogonale Funktion bereitgestellt wird und durch Anfitten der orthogonalen Funktion an den Verlauf des demodulierten Messsignals eine Orthogonal-Komponente des Messergebnisses erzeugt wird,
- zur Messkalibrierung bei bekannter Konzentration der zu messenden Gaskomponente ein Störparameter variiert und dabei ein Fehler ermittelt wird, der aus einer Inphase-Komponente in Form der Differenz zwischen dem erhaltenen Messergebnis und einem Soll-Messergebnis und der Orthogonal-Komponente besteht, wobei ferner ein Zusammenhang zwischen der Inphase-Komponente des Fehlers und seiner Orthogonal-Komponente ermittelt wird, und

- beim Messen einer unbekannten Konzentration der Gaskomponente das dabei erhaltene Messergebnis mit einer Inphase-Komponente korrigiert wird, die anhand des bei der Messkalibrierung ermittelten Zusammenhangs aus der ebenfalls erhaltenen Orthogonal-Komponente bestimmt wird.

Die zu der Sollkurve, d. h. zu dem im Idealfall zu erwartenden Verlauf des demodulierten Messsignals, orthogonale Funktion korreliert aufgrund ihrer orthogonalen Charakteristik nicht mit der Form der zu messenden Absorptionslinie (genauer dem Verlauf ihrer Demodulationsfrequenzkomponente). Stattdessen korreliert die orthogonale Funktion mit den Orthogonal-Komponenten von Störsignalen, deren Inphase-Komponenten ihrerseits unmittelbar als Störsignalanteile in dem Messsignal erscheinen.

Indem zusätzlich zur Sollkurve auch die zu ihr orthogonale Funktion an das demodulierte Messsignal angefittet wird, erhält man neben dem störungsbehafteten Messergebnis (genauer der störungsbehafteten Inphase-Komponente des Messergebnisses) auch eine Orthogonal-Komponente des Messergebnisses, die gleich der Orthogonal-Komponente der Störung ist. Im Rahmen einer Messkalibrierung kann daher bei bekannter Konzentration der zu messenden Gaskomponente ein Messfehler mit einer Inphase- und einer Orthogonal-Komponente ermittelt werden, wobei die Inphase-Komponente des Fehlers aus der Differenz zwischen dem erhaltenen Messergebnis und einem Sollmessergebnis der bekannten Konzentration besteht. Nach Ermittlung eines Zusammenhangs zwischen der Inphase- und der Orthogonal-Komponente des Fehlers kann daher beim Messen einer unbekannten Konzentration der Gaskomponente das dabei erhaltene Messergebnis unter Verwendung des genannten Zusammenhangs und der ebenfalls erhaltenen Orthogonal-Komponente korrigiert werden.

Wie oben bereits erwähnt, kann die Störung bzw. der Fehler zu einem großen Anteil temperaturabhängig sein. Daher kann während der Kalibrierung die Betriebstemperatur des Gasanalysators variiert werden, so dass der ermittelte Fehler temperaturabhängig ist bzw. sich im Verlauf der Erwärmung oder Abkühlung des Gasanalysators ändert. Etaloneffekte im optischen Strahlengang führen dazu, dass sich der Fehler über den Temperaturverlauf periodisch ändert, d. h. der von der Inphase- und der Orthogonal-Komponente des Fehlers gebildete Vektor rotiert. Andere Störeffekte, wie z. B. die Änderung der Reflektivität eines Etalons, führen dagegen zu Längenänderungen des Fehlervektors und/oder Offsets der Inphase- und Orthogonal-Komponente. Es ist daher möglich, aufgrund des Fehlerverlaufs, also der Art und Weise, wie sich der Fehler verändert, zwischen unterschiedlichen Störungsarten zu unterscheiden.

Da, wie bereits erwähnt, Etaloneffekte zu einer periodischen Änderung oder Schwankung des Fehlers über den Temperaturverlauf führen, so dass der Fehlervektor rotiert, ist der zu ermittelnde Zusammenhang zwischen der Inphase- und der Orthogonal-Komponente des Fehlers nicht eindeutig. Daher wird bei der Messkalibrierung vorzugsweise auch die Temperatur gemessen, um einen funktionellen und somit eindeutigen Zusammenhang zwischen der Inphase-Komponente des Fehlers, seiner Orthogonal-Komponente und der gemessenen Temperatur ermitteln zu können.

Alternativ oder ergänzend kann auch der Fehlereinfluss einer Störgaskomponente in dem Messgas kompensiert werden, die sich spektral mit der interessierenden Absorptionslinie der zu messenden Gaskomponente überlagert. In diesem Fall wird der Gasanalysator bei unterschiedlichen bekannten Konzentrationen der Störgaskomponente kalibriert und dabei ein funktioneller Zusammenhang zwischen der Inphase-Komponente des Fehlers, seiner Orthogonal-Komponente und der Konzentration der Störgaskomponente ermittelt. Zwar könnte die Störgaskomponente auf die gleiche Weise wie die zu messende Gaskomponente durch Anfitten einer entsprechenden Sollkurve an den Verlauf des demodulierten Messsignals ermittelt werden, jedoch würde sich dabei umgekehrt die zu messende Gaskomponente störend bemerkbar machen. Demgegenüber hat das erfindungsgemäße Verfahren den Vorteil, dass die interessierende Gaskomponente keinen Einfluss auf die Störgasmessung hat. Weiterhin wird die Störgasmessung auf den spektral- und frequenzrelevanten Anteil beschränkt, was verhindert, dass weitere Einflussgrößen die Störgasmessung beeinflussen, wie z. B. eine dritte Gaskomponente, die sich spektral nur mit der Störgaskomponente, nicht jedoch mit der zu messende Gaskomponente überlagert.

Zur weiteren Erläuterung der Erfindung wird im Folgenden auf die Figuren der Zeichnung Bezug genommen; im Einzelnen zeigen:

Figur 1    ein Ausführungsbeispiel eines Gasanalysator zur Durchführung des erfindungsgemäßen Verfahrens,

Figur 2    ein Beispiel für die Störung eines demodulierten Messsignals,

Figur 3    ein Beispiel für eine dem idealerweise zu erwartenden Verlauf des demodulierten Messsignals entsprechende Sollkurve und eine dazu orthogonale Funktion,

Figur 4    ein Beispiel für ein durch Anfitten der Sollkurve und der orthogonalen Funktion an das demodulierte Messsignal erhaltenes temperaturabhängiges Messergebnis, und

Figur 5 ein Beispiel für Änderungen des Messergebnisses innerhalb unterschiedlicher Temperaturbereiche.

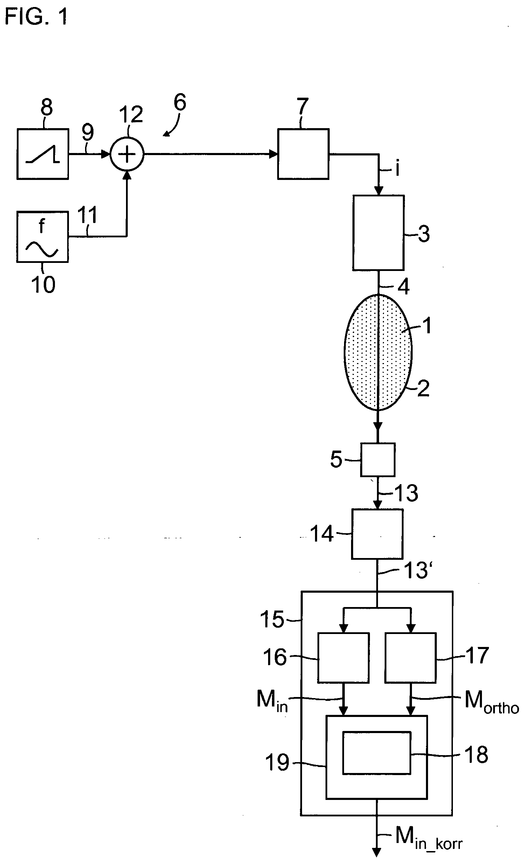

Bei dem in Figur 1 in Form eines vereinfachten Blockschaltbildes gezeigten Gasanalysator handelt es sich um ein Laserspektrometer zur Messung der Konzentration mindestens einer interessierenden Gaskomponente eines Messgases 1, das in einem Messvolumen 2, beispielsweise einer Messküvette oder einer Prozessgasleitung, enthalten ist. Das Spektrometer enthält eine Lichtquelle 3 in Form einer Laserdiode, deren Licht 4 nach Durchstrahlen des Messgases 1, auf einen Messdetektor 5 fällt. Eine von einer Modulationseinrichtung 6 gesteuerte Stromquelle 7 speist die Laserdiode 3 mit einem Injektionsstrom i, wobei die Intensität und Wellenlänge des erzeugten Lichts 4 von dem Strom i und der Betriebstemperatur der Laserdiode 3 abhängen. Die Modulationseinrichtung 6 umfasst einen ersten Signalgenerator 8, der die Stromquelle 7 periodisch mit einer vorgegebenen, vorzugsweise rampen- oder dreieckförmigen Funktion 9 angesteuert, um mit der mehr oder weniger linear dem Verlauf des Stromes i folgenden Wellenlänge des erzeugten Lichts 4 eine ausgewählte Absorptionslinie der interessierenden Gaskomponente abzutasten. Ein zweiter Signalgenerator 10 erzeugt ein sinusförmiges Signal 11 höherer Frequenz $f_0$, mit dem in einem Summierglied 12 die rampen- oder dreieckförmige Funktion 9 moduliert wird.

Der Messdetektor 5 erzeugt in Abhängigkeit von der detektierten Lichtintensität ein Messsignal 13, das in einem Lock-in-Verstärker 14 bei einer Harmonischen $nf_0$ (n = 1, 2, 3...), hier z. B. $2f_0$, der Modulationsfrequenz $f_0$ demoduliert wird. In einer nachgeordneten einer Auswerteeinrichtung 15 wird das demodulierte Messsignal 13' für jedes Abtastintervall zu einem Messergebnis ausgewertet. Dazu werden in einer ersten Recheneinheit 16 eine dem idealen demodulierten Messsignal 13' entsprechende Sollkurve und in einer zweiten Recheneinheit 17 eine zu der Sollkurve orthogonale Funktion an das demodulierte Messsignal 13' angefittet.

Wie eingangs bereits erläutert, können Temperaturänderungen innerhalb des Gasanalysators zu einer Drift der Messergebnisse führen, wobei eine Ursache für die Drift Etalons im optischen Strahlengang sind, die zu periodischen Strukturen im Verlauf des demodulierten Messsignals 13' führen.

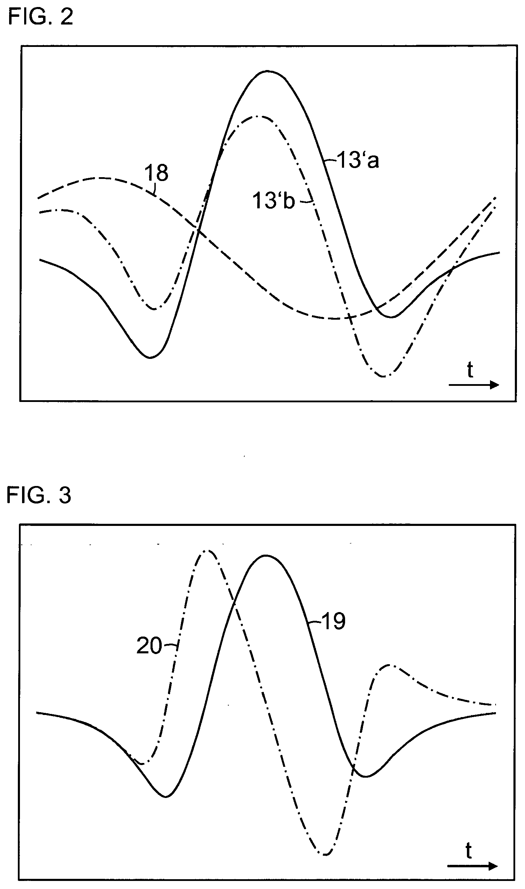

Figur 2 zeigt beispielhaft ein bei der zweiten Harmonischen $2f_0$ der Modulationsfrequenz $f_0$ demoduliertes störungsfreies Messsignal 13'a, eine periodische Störung 18 sowie das von der Störung 18 überlagerte Messsignal 13'b. Es ist sofort erkennbar, dass ein Anfitten einer dem idealen demodulierten Messsignal 13'a entsprechende Sollkurve (19 in Figur 3) an das gestörte Messsignal 13'b nicht zu einer korrekten Konzentrationsbestimmung führt.

Figur 3 zeigt beispielhaft die dem idealerweise zu erwartenden Verlauf des demodulierten Messsignals 13' entsprechende Sollkurve 19 und eine dazu orthogonale Funktion 20. Im Unterschied zu der Sollkurve 19 korreliert die orthogonale Funktion 20 nicht mit dem demodulierten Messsignal 13' bzw. 13'a, sondern mit den Orthogonal-Komponenten von Störsignalen, deren Inphase-Komponenten ihrerseits unmittelbar als Störsignalanteile in dem demodulierten Messsignal 13' bzw. 13'a erscheinen.

Zurück zu Figur 1 liefert die Recheneinheit 16 ein mehr oder weniger gestörtes Messergebnis M, genauer eine Inphase-Komponente $M_{in}$ des Messergebnisses M bestehend aus einem Nutzanteil $S = S_{in}$ und einem Inphase-Störanteil $N_{in}$. Die Recheneinheit 17 erzeugt eine Orthogonal-Komponente $M_{ortho}$ des Messergebnisses M bestehend aus dem Orthogonal-Störanteil $N_{ortho}$:

$$M = M_{in} + M_{ortho} = (S + N_{in}) + N_{ortho} \text{ mit } S = S_{in} \text{ und } S_{ortho} = 0.$$

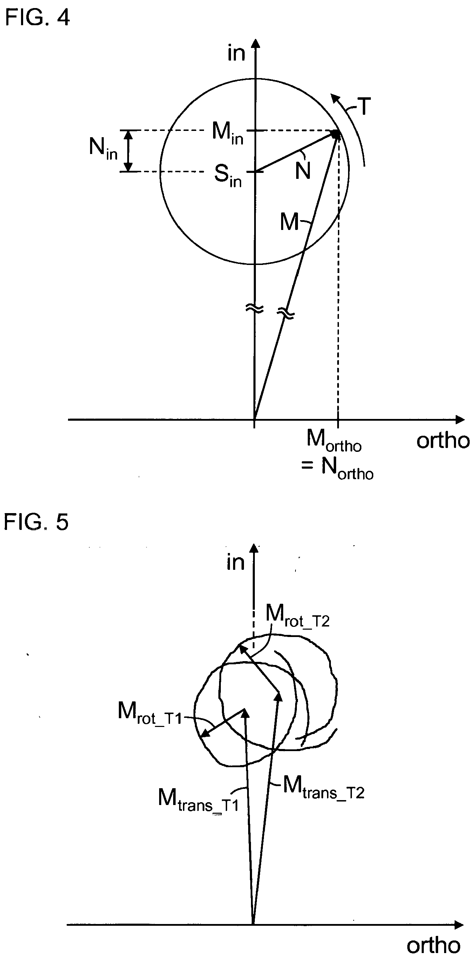

Figur 4 zeigt beispielhaft ein Zeigerdiagramm des Messergebnisses M mit seinen Inphase- und Orthogonal-Komponenten. Zur Messkalibrierung wird bei bekannter Konzentration der zu messenden Gaskomponente die Betriebstemperatur des Gasanalysators variiert und dabei ein Fehler N ermittelt, der aus der Inphase-Komponente $N_{in}$ in Form der Differenz zwischen dem Inphase-Messergebnis $M_{in}$ und dem Soll-Messergebnis $S_{in}$ und aus der Orthogonal-Komponente $M_{ortho}$ besteht. In einen nächsten Schritt wird ein Zusammenhang zwischen der Inphase-Komponente $N_{in}$ des Fehlers N und seiner Orthogonal-Komponente $N_{ortho}$ ermittelt. Figur 4 zeigt einen einfachen Fall, bei dem der auf Etaloneffekten beruhende Fehler N über die Temperatur T in etwa einen Kreis um das Soll-Messergebnis $S_{in}$ beschreibt. Dieser Zusammenhang ist in einem Speicher 18 (Figur 1) einer den Recheneinheiten 16, 17 nachgeordneten weiteren Recheneinheit 19 abgespeichert, die beim Messen einer unbekannten Konzentration der Gaskomponente das dabei erhaltene Inphase-Messergebnis $M_{in}$ mit einer Inphase-Komponente korrigiert, die unter Verwendung des abgespeicherten Zusammenhangs aus der ebenfalls erhaltenen Orthogonal-Komponente $M_{ortho}$ bestimmt wird. Bei dem in Figur 4 gezeigten einfachen Beispiel wird im Rahmen der Messkalibrierung der Radius R = |N| des Kreises bestimmt und beim Messen einer unbekannten Konzentration der Gaskomponente das dabei erhaltene Inphase-Messergebnis $M_{in}$ wie folgt korrigiert: $Min\_korr = M_{in} \pm (R^2 - M^2_{ortho})^{1/2}$.

Figur 5 zeigt ein Beispiel für Änderungen des Messergebnisses M innerhalb unterschiedlicher Temperaturbereiche T1 und T2, z. B. T1 = 45°C bis 49°C und T1 = -6°C bis -2°C. In der Messrealität sind unter Umständen mehrere Etalons mit verschiedenen Temperaturabhängigkeiten und -empfindlichkeiten wirksam, so dass die von ihnen verursachten Fehler in dem Zeigerdiagramm mit unterschiedlichen Geschwindigkeiten rotieren können. Weiterhin können sich die Amplituden (Zeigerlängen) aufgrund von Änderungen der Intensität (Alignmentabhängigkeit der Temperatur) oder durch Änderungen der Reflektivitäten (z. B. Beschichtungen optischer Flächen) ändern. Die Amplituden ändern sich auch dann, wenn andere Störeinflüsse hinzukommen, die sich nicht wie Etalons verhalten. Die Messergebnisse M(T1), M(T2) bestehen dann aus Komponenten $M_{trans\_T1}$, $M_{trans\_T2}$, die sich in dem Zeigerdiagramm in Abhängigkeit von der Temperatur translatorisch bewegen, und solche, $M_{rot\_T1}$, $M_{rot\_T2}$, die sich rotatorisch bewegen.

## Patentansprüche

1. Verfahren zur Messung der Konzentration einer Gaskomponente in einem Messgas (1) mittels eines Gasanalysators, wobei

   - zur wellenlängenabhängigen Abtastung einer interessierenden Absorptionslinie der Gaskomponente die Wellenlänge des Lichts (4) einer wellenlängenabstimmbaren Lichtquelle (3) innerhalb periodisch aufeinanderfolgender Abtastintervalle variiert und dabei zusätzlich mit einer Frequenz ($f_0$) moduliert wird,
   - das modulierte Licht (4) durch das Messgas (1) auf einen Detektor (5) geführt wird,
   - ein von dem Detektor (5) erzeugtes Messsignal (16) bei einer Oberschwingung ($nf_0$) der Frequenz ($f_0$) demoduliert wird, und
   - durch Anfitten einer Sollkurve (19) an den Verlauf des demodulierten Messsignals (13') ein Messergebnis erzeugt wird,

   **dadurch gekennzeichnet,**

   - **dass** eine zu der Sollkurve (19) orthogonale Funktion (20) bereitgestellt wird und durch Anfitten der orthogonalen Funktion (20) an den Verlauf des demodulierten Messsignals (13') eine Orthogonal-Komponente ($M_{ortho}$) des Messergebnisses erzeugt wird,
   - **dass** zur Messkalibrierung bei bekannter Konzentration der zu messenden Gaskomponente ein Störparameter variiert und dabei ein Fehler (N) ermittelt wird, der aus einer Inphase-Komponente ($N_{in}$) in Form der Differenz zwischen dem erhaltenen Messergebnis ($M_{in}$) und einem Soll-Messergebnis ($S_{in}$) und der Orthogonal-Komponente ($M_{ortho}$) besteht, wobei ferner ein Zusammenhang zwischen der Inphase-Komponente ($N_{in}$) des Fehlers (N) und seiner Orthogonal-Komponente ($M_{ortho}$) ermittelt wird, und
   - **dass** beim Messen einer unbekannten Konzentration der Gaskomponente das dabei erhaltene Messergebnis ($M_{in}$) mit einer Inphase-Komponente korrigiert wird, die anhand des bei der Messkalibrierung ermittelten Zusammenhangs aus der ebenfalls erhaltenen Orthogonal-Komponente ($M_{ortho}$) bestimmt wird.

2. Verfahren nach Anspruch 1, **dadurch gekennzeichnet, dass** der bei der Messkalibrierung ermittelte Zusammenhang in Form eines funktionellen Zusammenhang zwischen der Inphase-Komponente ($N_{in}$) des Fehlers (N), seiner Orthogonal-Komponente ($N_{ortho}$) und des gemessenen oder bekannten Störparameters ermittelt wird.

3. Verfahren nach Anspruch 1 oder 2, **dadurch gekennzeichnet, dass** als Störparameter die Betriebstemperatur (T) des Gasanalysators variiert wird.

4. Verfahren nach Anspruch 1 oder 2, **dadurch gekennzeichnet, dass** als Störparameter die Konzentration einer Störgaskomponente in dem für die Kalibration verwendeten Messgas variiert wird.

## Claims

1. Method for measuring the concentration of a gas component in a measurement gas (1) by means of a gas analyzer, in which

   - for the purpose of wavelength-dependent scanning of a gas component absorption line of interest, the wavelength of the light (4) of a wavelength-tunable light source (3) is varied within periodically consecutive scan intervals and is additionally modulated in the process with a frequency ($f_0$),
   - the modulated light (4) is guided through the measurement gas (1) onto a detector (5),

- a measurement signal (16) generated by the detector (5) is demodulated in the event of a harmonic ($nf_0$) of the frequency ($f_0$), and
- a measurement result is produced by fitting a desired curve (19) to the profile of the demodulated measurement signal (13'),

**characterized in that**

- a function (20) orthogonal to the desired curve (19) is provided, and an orthogonal component ($M_{ortho}$) of the measurement result is produced by fitting the orthogonal function (20) to the profile of the demodulated measurement signal (13'),
- for the purpose of measurement calibration given a known concentration of the gas component to be measured, an interfering parameter is varied and there is determined in the process an error (N) which consists of an inphase component ($N_{in}$), in the form of the difference between the obtained measurement result ($M_{in}$) and a desired measurement result ($S_{in}$), and of the orthogonal component ($M_{ortho}$) a relationship between the inphase component ($N_{in}$) of the error (N) and its orthogonal component ($M_{ortho}$) further being determined, and
- in the case of the measurement of an unknown concentration of the gas component the measurement result ($M_{in}$) obtained in the process is corrected with an inphase component which is determined, with the aid of the relationship determined in the measurement calibration, from the likewise obtained orthogonal component ($M_{ortho}$).

2. Method according to Claim 1, **characterized in that** the relationship determined in the measurement calibration is determined in the form of a functional relationship between the inphase component ($N_{in}$) of the error (N), its orthogonal component ($M_{ortho}$) and the measured or known interfering parameter.

3. Method according to Claim 1 or 2, **characterized in that** it is the operating temperature (T) of the gas analyzer that is varied as interfering parameter.

4. Method according to Claim 1 or 2, **characterized in that** it is the concentration of an interfering gas component in the measurement gas used for the calibration that is varied as interfering parameter.

**Revendications**

1. Procédé de mesure de la concentration d'un constituant gazeux d'un gaz ( 1 ) au moyen d'un analyseur de gaz dans lequel

- pour la détection en fonction de la longueur d'onde d'une raie d'absorption, à laquelle on s'intéresse, du constituant gazeux, on fait varier la longueur d'onde de la lumière ( 4 ) d'une source ( 3 ) lumineuse pouvant être définie en longueur d'onde dans des intervalles de détection se succédant périodiquement, et on la module supplémentairement à une fréquence ( $f_0$ ),
- on envoie la lumière ( 4 ) modulée par le gaz ( 1 ) sur un détecteur ( 5 ),
- on démodule le signal ( 16 ) de mesure produit par le détecteur ( 5 ) à un harmonique ( $nf_0$ ) de la fréquence ( $f_0$ ) et
- en ajustant une courbe ( 19 ) de consigne à la variation du signal ( 13 ) de mesure démodulé, on produit un résultat de mesure,

**caractérisé**

- **en ce qu'**on se procure une fonction ( 20 ) orthogonale à la courbe ( 19 ) de consigne et en ajustant la fonction ( 20 ) orthogonale à la variation du signal ( 13' ) de mesure démodulé, on produit une composante ( $M_{ortho}$ ) orthogonale du résultat de mesure,
- **en ce que**, pour l'étalonnage de mesure, on fait varier, pour une concentration connue du constituant gazeux à mesurer, un paramètre de perturbation et on détermine ainsi une erreur ( N ), qui est constituée d'une composante ( $N_{in}$ ) en phase, sous la forme de la différence entre le résultat ( $M_{in}$ ) de mesure obtenu et un résultat ( $S_{in}$ ) de mesure de consigne et la composante ( $M_{ortho}$ ) orthogonale, une relation entre la composante ( $N_{in}$ ) en phase de l'erreur ( N ) et sa composante ( $M_{ortho}$ ) orthogonale étant en outre déterminée et
- **en ce que**, lors de la mesure d'une concentration inconnue du constituant gazeux, on corrige le résultat ( $M_{in}$ ) de mesure obtenu par une composante en phase, qui est déterminée à partir de la composante ( $M_{ortho}$ )

orthogonale obtenue également, à l'aide de la relation déterminée lors de l'étalonnage de mesure.

2.  Procédé suivant la revendication 1, **caractérisé en ce que** l'on détermine la relation déterminée lors de l'étalonnage de mesure sous la forme d'une relation fonctionnelle entre la composante ( $N_{in}$ ) en phase de l'erreur ( $N$ ), sa composante ( $N_{ortho}$ ) et le paramètre de perturbation mesuré ou connu.

3.  Procédé suivant la revendication 1 ou 2, **caractérisé en ce qu'**on fait varier comme paramètre de perturbation la température ( $T$ ) de fonctionnement de l'analyseur de gaz.

4.  Procédé suivant la revendication 1 ou 2, **caractérisé en ce que** l'on fait varier comme paramètre de perturbation la concentration d'un constituant gazeux de perturbation dans le gaz utilisé pour l'étalonnage.

FIG. 1

FIG. 2

FIG. 3

FIG. 4

FIG. 5

**IN DER BESCHREIBUNG AUFGEFÜHRTE DOKUMENTE**

**In der Beschreibung aufgeführte Patentdokumente**

- EP 1475618 B1
- EP 2336738 A1

- EP 1927831 A1